# EUROPEAN PATENT APPLICATION

(11) **EP 4 109 077 A1**
(43) Date of publication of application: **28.12.2022**
(21) Application number: 21756900.3
(22) Date of filing: 13.01.2021
(51) Int. Cl.: G01N 21/64, G01N 33/543, G01N 21/63

(54) **CHROMATOGRAPHIC TESTING DEVICE CONFIGURED TO SELECT IMAGING SCHEME ACCORDING TO TYPE OF FLUORESCENT DYE, AND METHOD FOR CONTROLLING SAME**

(30) Priority: 21.02.2020 KR 20200021787
(71) Applicant: OSANG HEALTHCARE CO., LTD., Anyang-si, Gyeonggi-do 14040 (KR)
(72) Inventor: SHIN, Dae Sup, Seoul 01223 (KR); SHIM, Dong Whi, Incheon 21584 (KR); CHUNG, Hyun Woo, Seoul 01858 (KR); HONG, Ju Pyo, Seoul 02517 (KR); KO, Hee Young, Sokcho-si Gangwon-do 24848 (KR)
(74) Representative: AWA Sweden AB
(86) International application number: PCT/KR2021/000473
(87) International publication number: WO 2021/167248

(57) **Abstract**

The present invention relates to a chromatographic testing device which is configured to select an imaging scheme according to the type of fluorescent dye in an immunochromatographic test cartridge, and a method for controlling the same. One aspect of the present invention provides a chromatographic testing device comprising: a body; a code expression recognition unit provided in the body and recognizing a code expression in which a type of fluorescent dye of an immune strip provided on an immunochromatographic test cartridge mounted on the body is recorded; an excitation light source emission unit for emitting light from an excitation light source to the immunochromatographic test cartridge; an image sensor unit for recognizing excitation light generated by the excitation light source; and a control unit for controlling the image sensor unit in a single imaging scheme or a cumulative light imaging scheme according to the type of fluorescent dye of an immune strip, recognized by the code expression recognition unit.

## Description

### CROSS-REFERENCE TO RELATED APPLICATION

This application claims priority to and the benefit of Korean Patent Application No. 10-2020-0021787, filed on February 21, 2020, the disclosure of which is incorporated herein by reference in its entirety.

### [Technical Field]

The present invention relates to a chromatographic testing device and a method for controlling the same, and more specifically to a chromatographic testing device configured to select an imaging scheme according to the type of fluorescent dye in an immunochromatographic test cartridge and a method for controlling the same.

### [Background Art]

The immune chromatography test, also known as the rapid test, is a method that can qualitatively and quantitatively test a trace amount of analyte in a short time by using an antigen-antibody reaction, and it is used in various fields such as medicine, agriculture, food, environment and the like, as well as testing for various diseases.

In this immune chromatography test, a sample such as blood, body fluid or the like is applied to a cartridge equipped with an analysis strip coated or impregnated with an antigen material that reacts with a specific antibody in the sample, and then the presence or absence of the antibody is determined through the reaction result of the analysis strip.

In addition, when the amount of antibody is small, the antigen-antibody reaction may also be weak, and in order to further maximize this, it can be identified using a fluorescence reaction or the like.

A chromatographic testing device is provided to perform an immunochromatographic test through the fluorescence reaction.

The chromatographic testing device can determine the presence and extent of antigens such as whether infection occurred by analyzing the fluorescence reaction (excitation light) generated by emitting light to an analysis strip to which the sample is applied.

Meanwhile, such a chromatographic test method is often manufactured based on biochemical materials such as immunological substances and the like, and due to the characteristics of the materials of these quantitative measurement biosensors, different signal reproducibility is obtained for each manufacturing lot, and accordingly, a code expression which is suitable for each lot is provided such that the deviation of signals between the lots can be corrected.

In addition, a fluorescent dye is applied to an analysis strip for the fluorescence reaction. As such a fluorescent dye, the time-resolved fluorescence (TRF) type fluorescent dye to which an organic fluorescent substance is applied is generally used.

Therefore, when the excitation light is received and analyzed through the fluorescence reaction of the fluorescent dye, it is necessary to increase the amount of light of the received excitation light in order to increase the accuracy of the test.

Further, in order to obtain a clearer image under the condition of the low amount of light of the excitation light in the TRF type, a method of increasing the irradiation intensity of the excitation light source or increasing the irradiation time, or increasing the exposure time of the image sensor receiving the excitation light or increasing the sensitivity has been proposed.

However, in the case of an organic fluorescent substance, since the organic fluorescent substance is characterized by decolorization depending on the intensity of the excitation light source, there is a limitation in increasing the intensity of the light source.

In addition, when the LED is used as an excitation light source, the heat generation of the LED is increased as the emission intensity or the emission time is increased, and since the surroundings of the LED and the image sensor are formed as dark chambers in order to prevent the light from being introduced from the outside, there is a problem in that it is difficult to ventilate and the heat dissipation is disadvantageous, and thus, the lifespan of the LED may be shortened.

In addition, there is also a problem in that the method of increasing the exposure time of the image sensor receiving the excitation light or increasing the sensitivity also increases the temperature of the image sensor or increases the noise.

Further, in order to increase the intensity of the excitation light, the quantum dot (QD) type to which a quantum dot is applied instead of the organic fluorescent substance has also been proposed. However, since the TRF type and the QD type are mixedly used, it is necessary to distinguish which type of the fluorescent dye of the immunochromatographic test cartridge mounted on the chromatographic testing device is used.

### [Disclosure]

### [Technical Problem]

The present invention has been devised to solve the aforementioned problems, and it is an object of the present invention to provide a chromatographic testing device, which is capable of automatically changing an imaging scheme according to a fluorescent dye applied to an immunochromatographic test cartridge, and a method for controlling the same.

It is another object of the present invention to provide a chromatographic testing device, which is capable of automatically photographing by an appropriate imaging scheme according to a fluorescent dye applied to an immunochromatographic test cartridge, and a method for controlling the same.

In addition, it is still another object of the present invention to provide a chromatographic testing device, which is capable of obtaining a clearer excitation light image to obtain a more accurate test result, and a method for controlling the same.

The problems of the present invention are not limited to the above-described problems, and other problems that are not mentioned will be clearly understood by those skilled in the art from the following description.

### [Technical Solution]

In order to solve the aforementioned problems, according to an aspect of the present invention, a chromatographic testing device may be disclosed, which includes a body, a code expression recognition unit provided in the body and recognizing a code expression in which a type of fluorescent dye of an immune strip provided on an immunochromatographic test cartridge mounted on the body is recorded, an excitation light source emission unit for emitting light from an excitation light source to the immunochromatographic test cartridge, an image sensor unit for recognizing excitation light generated by the excitation light source, and a control unit for controlling the image sensor unit in a single imaging scheme or a cumulative light imaging scheme according to the type of fluorescent dye of an immune strip, recognized by the code expression recognition unit.

The code expression recognition unit may determine whether the fluorescent dye of the immunochromatographic test cartridge mounted on the body is a time resolved fluorescence (TRF) or a quantum dot through the code expression recognition.

The control unit may control the image sensor unit to photograph in a cumulative light imaging scheme, when the fluorescent dye of the immune strip recognized by the code expression recognition unit is a TRF, and control the image sensor unit to photograph in a single imaging scheme, when the fluorescent dye of the immune strip recognized by the code expression recognition unit is a quantum dot.

In the cumulative light imaging scheme, the excitation light source emission unit may emit light multiple times for a set number of times, and the image sensor unit may be exposed to excitation light multiple times each time the excitation light source emission unit emits light, and the amount of light detected each time it is exposed to the excitation light may be accumulated and imaged as an amount of light accumulated in the image sensor unit.

In the single imaging scheme, the excitation light source emission unit may emit light a single time, and when the excitation light source emission unit emits light, the excitation light may be received by the image sensor unit and imaged.

The code expression recognition unit may include a code expression illumination light source for illuminating the code expression, and the image sensor unit may receive light emitted from the code expression illumination light source and reflected in the code expression, and the control unit may read the code expression information.

Meanwhile, according to another aspect of the present invention, provided is a method for controlling a chromatographic testing device, including a code expression recognition step of recognizing a code expression of an immunochromatographic test cartridge mounted on a body, a determination step of determining the type of fluorescent dye of the immunochromatographic test cartridge recognized in the code expression recognition step, an imaging scheme selection step of selecting an excitation light imaging scheme according to the type of fluorescent dye determined in the determination step, and an imaging step of photographing excitation light according to the imaging scheme selected in the imaging scheme selection step.

The determination step may determine whether the fluorescent dye of the immunochromatographic test cartridge is a time resolved fluorescence (TRF) or a quantum dot.

When the fluorescent dye determined in the determination step is a time resolved fluorescence (TRF), a cumulative light imaging scheme may be selected in the imaging scheme selection step.

When the fluorescent dye determined in the determination step is a quantum dot, a single imaging scheme may be selected in the imaging scheme selection step.

The cumulative light imaging scheme may include an excitation light source emission step in which an excitation light source emits light, an excitation light emission step in which excitation light is emitted from the immunochromatographic test cartridge by an excitation light source, a light amount accumulation step of accumulating an amount of light received while an image sensor unit is exposed to receive the excitation light, after a background noise of the excitation light generated in the excitation light emission step is reduced, and a cumulative light amount imaging step of generating an image as an amount of light accumulated in the image sensor unit during which the excitation light source emission step, the excitation light emission step and the light amount accumulation step are repeated for a set number of times.

The single imaging scheme may include an excitation light source emission step in which an excitation light source emits light, an excitation light emission step in which excitation light is emitted from the immunochromatographic test cartridge by an excitation light source, a sensor exposure step in which the image sensor unit is exposed to receive the excitation light, and a single light amount imaging step of generating an image with the amount of light received in the sensor exposure step.

The method may further include a blood injection step for dropping a sample into the immunochromatographic test cartridge, and an excitation position moving step for moving the immunochromatographic test cartridge on which the blood injection has been completed to an excitation position for reading excitation light.

### [Advantageous Effects]

According to the chromatographic testing device for selecting an imaging scheme according to the type of fluorescent dye of the present invention and a method for controlling the same, the following effects are obtained.

First, there is an effect that the code expression of the cartridge may be automatically recognized and photographed by an optimal imaging scheme for the fluorescent dye corresponding to the corresponding code expression, thereby improving accuracy and convenience.

Second, since the code expression of the cartridge may be automatically recognized while minimizing additional components, there is an effect that the size may be reduced and the cost may be reduced.

Third, since the amount of light may be accumulated and photographed even for the cartridge to which the TRF-type fluorescent dye having a low amount of light of excitation light reaction is applied, there is an effect that a clearer excitation light image may be obtained, thereby improving accuracy.

The effects of the present invention are not limited to the above-mentioned effects, and other effects that are not mentioned may be clearly understood by those skilled in the art from the description of the claims.

### [Description of Drawings]

The summary set forth above as well as the detailed description of the preferred exemplary embodiments of the present application set forth below may be better understood when read in conjunction with the accompanying drawings. For the purpose of illustrating the present invention, preferred exemplary embodiments are illustrated in the drawings. It should be understood, however, that the present application is not limited to the precise arrangements and instrumentalities illustrated therein.
FIG. 1 is a perspective view illustrating the chromatographic testing device according to an exemplary embodiment of the present invention.
FIG. 2 is a perspective view illustrating a cartridge tray and a tray driving unit of the chromatographic testing device of FIG. 1.
FIG. 3 is a perspective view illustrating an immunochromatographic test cartridge.
FIG. 4 is a perspective view illustrating an image sensor unit, a code expression illumination light source and an excitation light source emission unit.
FIG. 5 is a graph illustrating the emission of an excitation light source and excitation light, the exposure time point of a sensor and the received amount of light, when the type of fluorescent dye is a time resolved fluorescence (TRF).
FIG. 6 a graph illustrating the emission of an excitation light source and excitation light, the exposure time point of a sensor and the received amount of light, when the type of fluorescent dye is a quantum dot.
FIG. 7 is a graph illustrating the emission of an excitation light source and excitation light, the exposure time point of a sensor and the received amount of light in a cumulative light imaging scheme.
FIG. 8 is a flowchart illustrating an exemplary embodiment of the method for controlling a chromatographic testing device according to the present invention.
FIG. 9 is a flowchart of an accumulative light imaging scheme.
FIG. 10 is a flowchart of a single imaging scheme.

### [Modes of the Invention]

Hereinafter, preferred exemplary embodiments of the present invention in which the objects of the present invention may be specifically implemented will be described with reference to the accompanying drawings. In the description of the present exemplary embodiment, the same names and the same reference numerals are used for the same components, and the additional description thereof will be omitted.

Hereinafter, an exemplary embodiment of the chromatographic testing device (hereinafter, referred to as 'a chromatographic testing device' for the convenience of description) that selects an imaging scheme according to the type of fluorescent dye according to the present invention will be described.

In the description of the chromatographic testing device 100 of the present exemplary embodiment, it will be described using blood or body fluid, saliva, feces and urine as an antibody sample as an example. However, the present invention is not necessarily limited thereto, and various samples other than blood or body fluid may be applied.

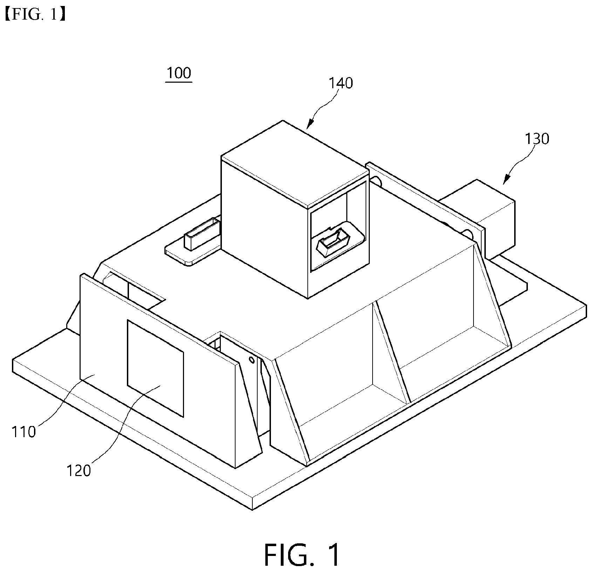

As illustrated in FIGS. 1, 2 and 4, the chromatographic testing device 100 according to the present exemplary embodiment may include a body 110, a cartridge tray 120, a tray driving unit 130, an image sensor unit 140, a code expression recognition unit 150, an excitation light source emission unit 160 and a control unit 180.

The body 110 forms an external shape, and a space for immunochromatographic analysis and a space in which parts are accommodated may be formed therein.

The cartridge tray 120 may be movably provided to be accommodated inside the body 110 or to be withdrawn to the outside of the body 110.

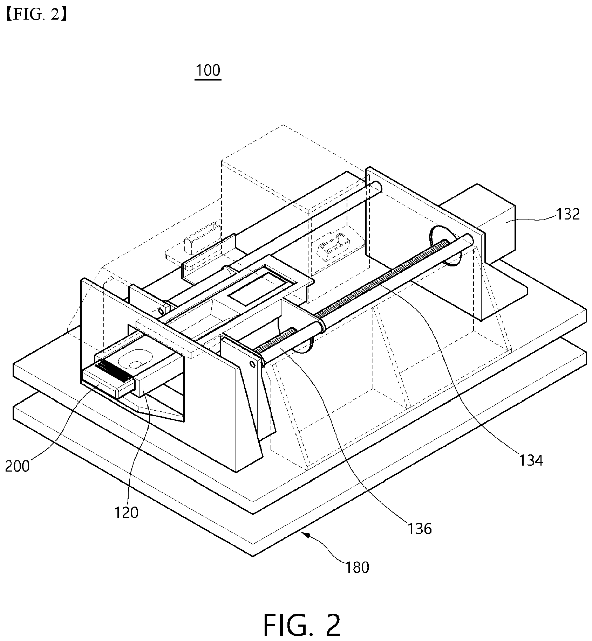

Meanwhile, a tray driving unit 130 for driving the cartridge tray 120 may be provided. As illustrated in FIG. 2, the tray driving unit 130 may include a motor 132 capable of rotating in the forward direction and rotating in the reverse direction under the control of the control unit 180, and a screw 134 rotated by the motor 132 and having a longitudinal direction parallel to the moving direction of the cartridge tray 120, and having a screw thread formed on the outer peripheral surface in the longitudinal direction. In addition, the screw 134 may be engaged with the cartridge tray 120 such that the cartridge tray 120 slides in one or the other direction according to the rotation of the screw 134. In addition, a guide 136 for guiding the movement of the cartridge tray 120 may be provided.

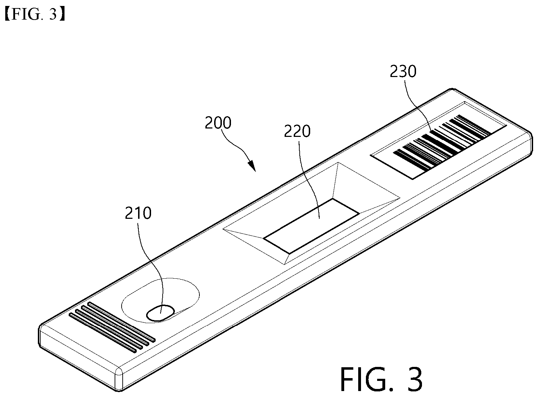

As illustrated in FIG. 3, the cartridge tray 120 may be provided such that the immunochromatography cartridge 200 (hereinafter, referred to as 'a cartridge 200' for the convenience of description) having an assay strip is inserted and mounted therein.

In addition, a rail (not illustrated) may be provided such that the cartridge 200 may be mounted in a sliding manner for more accurate and easy mounting. That is, the cartridge tray 120 may be provided with a rail (not illustrated) slidably coupled to the side of the cartridge 200 such that the cartridge 200 is mounted while being drawn in from the front in the horizontal direction. In this case, the rail (not illustrated) may be provided so as not to interfere with the upper surface of the cartridge 200 mounted on the cartridge tray 120.

As illustrated in FIG. 3, the cartridge 200 may include an immune strip inside a housing that forms an external shape.

In addition, an injection hole 210 for applying a sample such as blood or body fluid to the immune strip may be formed in the housing.

In addition, a window 220 may be formed to confirm the reaction of the immune strip to which the sample is applied or to confirm that the excitation light is emitted.

In addition, a code expression 230 in which information such as the type of marker such as a biosensor applied to a lot of the cartridge 200 or an immune strip may be printed or formed on one side of the housing

The code expression 230 may be formed of a barcode, a QR code or the like, but may also be provided as RFID or the like, and the present invention is not limited to the form of the code expression 230.

Meanwhile, a fluorescent dye is applied to the immune strip, and various types of fluorescent dyes may be applied as needed. As the fluorescent dye, the time resolved fluorescence (TRF) method to which an organic phosphor is applied may be applied, or the QD method to which a quantum dot is applied may be applied.

In addition, information on the method of a fluorescent dye applied to the immune strip may be written in the code expression 230.

Meanwhile, the image sensor unit 140 recognizes the code expression 230 of the cartridge 200, and in addition, it may be provided to confirm the excitation light reaction appearing in the immune strip of the cartridge 200 through a window 220.

The image sensor unit 140 may include an image sensor 142 for sensing image information and a filter 144 which is a component for filtering light received by the image sensor 142 so as not to pass through a specific wavelength band.

In addition, the image sensor unit 140 may include a lens group 146 for refracting the light received by the image sensor 142.

The filter 144 may be provided between the lens group 146 and the image sensor 142. Alternatively, the filter 144 may be provided on the front surface of the lens group 146.

In the present exemplary embodiment, the filter 144 is described as an example where the filter 144 is manufactured in a way that is fitted in the form of a cap on the lens group 146, but the present invention is not limited thereto, and the position of the filter 144 may be installed at a suitable position according to the needs of the manufacturer.

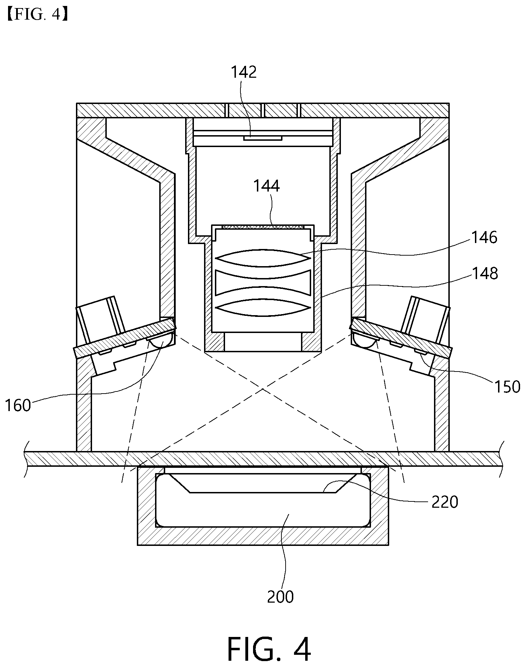

Meanwhile, as illustrated in FIG. 4, the code expression recognition unit 150 may determine whether the fluorescence dye of the cartridge 200 mounted on the cartridge tray is a time resolved fluorescence (TRF) or a quantum dot through the recognition of the code expression 230 formed in the cartridge 200.

As described above, the code expression 230 may be formed of a barcode, QR code, RFID or the like, and in the present exemplary embodiment, the code expression 230 will be described as an example in the form of a barcode that can be read by an optical method.

If the code expression 230 is made of a barcode or a QR code, the code expression recognition unit 150 for recognizing the code expression 230 should also have an optical structure, and when the code expression 230 is an electronic type such as an RFID or the like, it may be a reader for receiving the corresponding information. However, in the present exemplary embodiment, the case in which the code expression 230 is an optical type composed of a barcode or a QR code will be described as an example.

When the code expression 230 is an optical type composed of a barcode or a QR code, the code expression recognition unit 150 may include a light source such as an LED for illuminating the code expression of the cartridge 200.

In addition, the image sensor unit 140 receives the light emitted from the code expression recognition unit 150 and reflected from the code expression 230, and as the control unit 180 reads the information of the code expression 230 received from the image sensor unit 140, it may be determined whether the fluorescent dye of the cartridge 200 is a time resolved fluorescence (TRF) or a quantum dot.

As illustrated in FIG. 4, the light source of the code expression recognition unit 150 is provided around the image sensor 142, and it may be provided to illuminate a portion where the code expression 230 of the cartridge 200 is formed. The light source of the code expression recognition unit 150 may be provided as a light emitting diode (LED), and if necessary, other lighting means other than the LED may be provided.

In addition, as illustrated in FIG. 4, an excitation light source emission unit 160 may be provided. The excitation light source emission unit 160 is provided around the image sensor 142, and it may be provided to illuminate a portion of the window 220 of the cartridge 200.

The excitation light source emission unit 160 is an excitation light source for inducing an excitation light reaction of the immune strip of the cartridge 200 that has reacted with the antibody of the sample, and it may be provided to illuminate the window 220 of the cartridge 200. In this case, the excitation light source emission unit 160 may be applied with an LED similar to the code expression recognition unit 150, but is not limited thereto, and other types of light sources may be applied as needed.

Meanwhile, the code expression recognition unit 150 and the excitation light source emission unit 160 may be provided to emit light in different wavelength bands.

In this case, the excitation light generated from the window 220 of the cartridge 200 should be incident to the image sensor 142, not the light from the excitation light source emission unit 160, and to this end, this is because a filter 144 is provided to block light other than the wavelength band of the excitation light generated in the window 220 from being incident on the image sensor.

Accordingly, the light of the excitation light source emission unit 160 is not directly incident on the image sensor, and the excitation light of the cartridge 200 generated by being excited by the excitation light source emission unit 160 passes through the filter 144, and thus, light may be received by the image sensor 142.

In this case, since the light emitted from the code expression recognition unit 150 must be received by the image sensor 142 after being reflected by the code expression 230, the light emitted from the code expression recognition unit 150 may be light in a wavelength band that can pass through the filter 144.

Meanwhile, the excitation light source emission unit 160 may be provided on both sides of the image sensor 142. That is, a plurality of excitation light source emission units 160 may be provided to illuminate symmetrically and obliquely from the side toward the window 220 of the cartridge 200.

The reason why a plurality of excitation light source emission units 160 illuminate symmetrically and obliquely from the side is to uniformly irradiate the excitation light source without a shade on the analysis strip exposed by the window 220.

To this end, as illustrated in FIG. 4, the edge of the window 220 of the housing of the cartridge 200 may also be formed to be obliquely inclined to minimize a step difference.

In addition, the control unit 180 is a component for controlling the tray driving unit 130, the image sensor unit 140, the code expression recognition unit 150 and the excitation light source emission unit 160 described above, and it may be provided inside the main body 110. In addition, the control unit 180 may be provided to analyze information acquired through the image sensor unit 140 to determine whether the antigen is present in the sample and the degree thereof.

In addition, the control unit 180 may control the excitation light source emission unit 160 and the image sensor unit 140 according to the lot of the cartridge 200 and the type of the fluorescent dye recognized through the code expression 230.

That is, as information collected through the recognition of the code expression 230, optimal control corresponding to the corresponding lot and the type of the biomarker is performed through the lot of the cartridge 200 and the type of the biomarker.

For example, depending on whether the type of the fluorescent dye of the cartridge 200 recognized through the code expression 230 is the time resolved fluorescence (TRF) method using an organic phosphor or the quantum dot method using quantum dots, it is possible to control the emission of the excitation light source emission unit 160 and optimally control the analysis pattern of the excitation light recognized by the image sensor 142.

Hereinafter, this will be described in more detail.

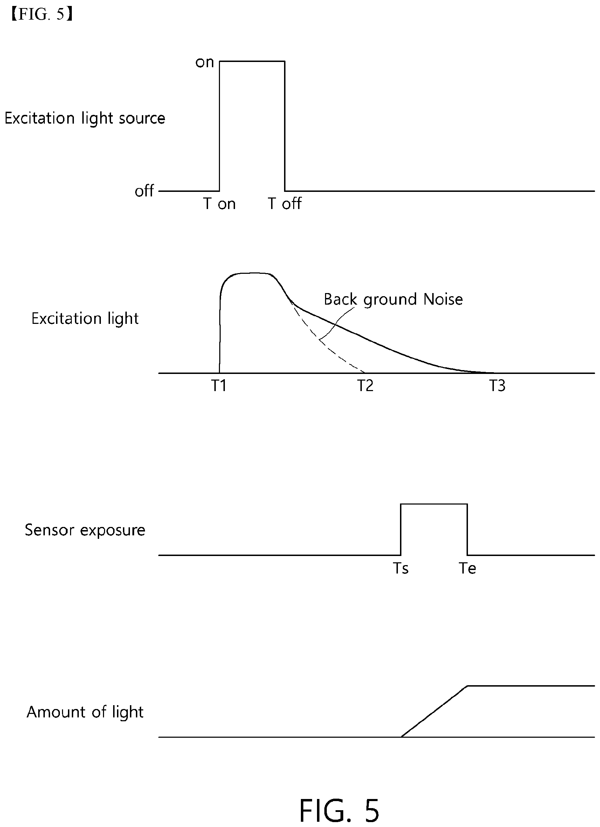

FIG. 5 is a graph illustrating the emission of an excitation light source and excitation light, the exposure time point of an image sensor unit 140 and the received amount of light, when the type of fluorescent dye is a time resolved fluorescence (TRF).

As illustrated in FIG. 5, the excitation light source may be emitted from a Ton time point to a Toff time point in the excitation light source emission unit 160.

In this case, in the immune strip of the cartridge 200, excitation light from an organic phosphor may be emitted at a time point T1 in response to the emitted excitation light source.

In this case, the excitation light is emitted at a time point T1 and gradually darkens until a time point T3, and in the excitation light, background noise due to impurities may also be emitted, and the emission of this background noise becomes dark faster than the excitation light and may be destroyed at a time point T2.

The image sensor unit 140 may receive the excitation light by starting exposure (Ts) at a time point T2 when the noise disappears, and ending the exposure (Te) at a time point T3 before the excitation light disappears.

In this case, the excitation light has the highest luminance at the time point T1, gradually decreases after Toff and then disappears at the time point T3, and thus, the amount of light received by the image sensor unit 140 is much lower than the maximum amount of light of the excitation light.

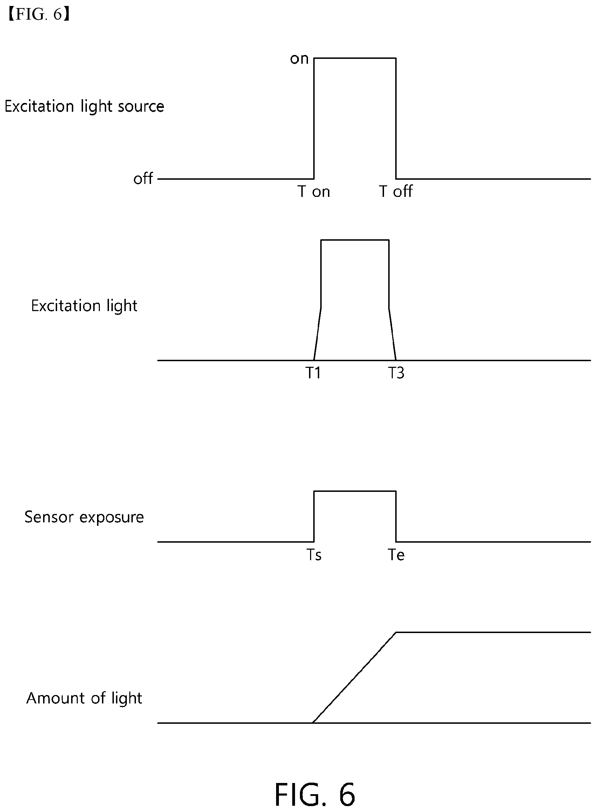

FIG. 6 a graph illustrating the emission of an excitation light source and excitation light, the exposure time point of an image sensor unit 140 and the received amount of light, when the type of fluorescent dye is a quantum dot.

As illustrated in FIG. 6, the excitation light source may be emitted from a time point Ton to a time point Toff in the excitation light source emission unit 160.

In addition, in the immune strip of the cartridge 200, light may be emitted at the time point T1 of the excitation light of the quantum dots in response to the emitted excitation light source.

In the case of the aforementioned TRF method, the excitation light is emitted at a time point T1 and gradually darkens until a time point T3, whereas in the case of the quantum dot method, the excitation light starts to emit at approximately the same time point as the Ton time point at which the excitation light source is emitted (T1), and the excitation light may disappear (T3) at about the same time point as the Toff time point when the excitation light source is turned off. For this reason, the exposure of the image sensor unit 140 also starts (Ts) at about the same time as the T1 time point at which the excitation light is emitted, and the exposure ends (Te) at about the same time as the T3 time point at which the excitation light disappears.

However, in the case of quantum dots, the degree of fluorescence reaction is very strong compared to organic phosphors. Further, in the case of the TRF method, exposure of the image sensor started after the noise disappeared, but in the case of quantum dots, exposure starts (Ts) at almost the same time as the T1 time point at which the excitation light is emitted, and since the exposure is terminated (Te) almost at the same time point as the T3 time point at which the excitation light disappears, the amount of light received by the image sensor unit 140 may be much larger than that of the TRF method.

Therefore, when the cartridge 200 recognized through the code expression 230 is the TRF method, the control unit 180 may control the image sensor unit to photograph in a cumulative light imaging scheme, and when the corresponding cartridge 200 is the quantum dot method, it may control the image sensor unit 140 to photograph in a single imaging scheme.

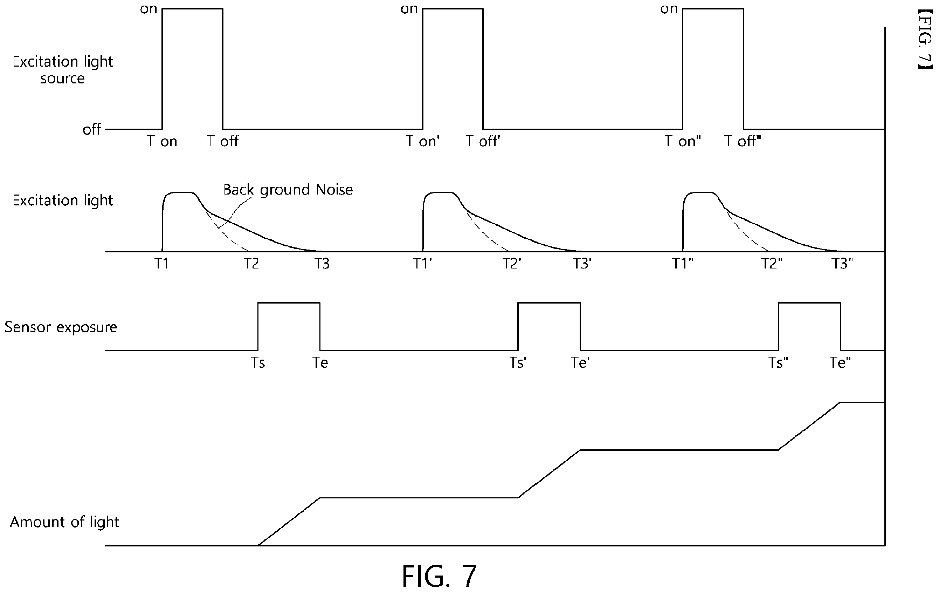

FIG. 7 is a graph illustrating the emission of an excitation light source and excitation light, the exposure time point of an image sensor unit 140 and the received amount of light for describing a cumulative light imaging scheme.

In the cumulative light imaging scheme, the excitation light source emits light multiple times for a set number of times, and whenever the excitation light source emits light, the image sensor unit 140 is exposed to the excitation light multiple times, and when exposed to the excitation light, it is a method of accumulating an amount of light detected for each time and imaging as the amount of light accumulated in the image sensor unit 140.

That is, similar to the above-described TRF method, the excitation light source emits light multiple times at regular intervals. In this case, the emission interval of the excitation light source may be longer than the time until the excitation light is emitted and then extinguished.

That is, when the excitation light source emits light from Ton to Toff, the excitation light may be emitted at a time point T1 and gradually darken until T3.

In this case, in the image sensor unit 140, exposure starts (Ts) at a time point T2 when the noise of the excitation light disappears, and exposure ends (Te) at a time point T3 before the excitation light disappears so as to receive light. Further, in this case, the amount of light may be accumulated in the form of electrons in the image sensor unit 140.

In addition, after the excitation light disappears, the excitation light source may emit light from Ton' to Toff. In addition, the excitation light may also be emitted from the excitation light at a time point T1' and gradually darken until T3, and in this case, in the image sensor unit 140, exposure starts (Ts') at a time point T2' when the noise of the excitation light disappears, and exposure ends (Te') at a time point T3' before the excitation light disappears so as to receive the excitation light.

In this case, the excitation light received by the image sensor unit 140 is accumulated in the form of electrons and may be accumulated together with electrons formed by the light received in the previous step.

In addition, similarly, after the excitation light disappears, the excitation light source may emit light from Ton" to Toff". Further, by this, the excitation light may also be emitted at a time point T1" and gradually darken until T3", and in this case, in the image sensor unit 140, exposure starts (Ts") at a time point T2" at which the noise of the excitation light disappears, and exposure ends at a time point T3" before the excitation light disappears (Te") so as to receive the excitation light.

In this case, the excitation light received by the image sensor unit 140 is accumulated in the form of electrons and may be accumulated together with electrons formed by the light received in the previous step.

Accordingly, as the emission of the excitation light source and the reception of the excitation light are repeated, electrons are accumulated in the image sensor unit 140, and a clearer image may be obtained in proportion to the repetition of the emission of the excitation light and the reception of the excitation light.

Hereinafter, the method for controlling a chromatographic testing device that selects an imaging scheme according to the type of fluorescent dye according to the present invention (hereinafter, referred to as 'a method for controlling a chromatographic testing device' for the convenience of description) will be described.

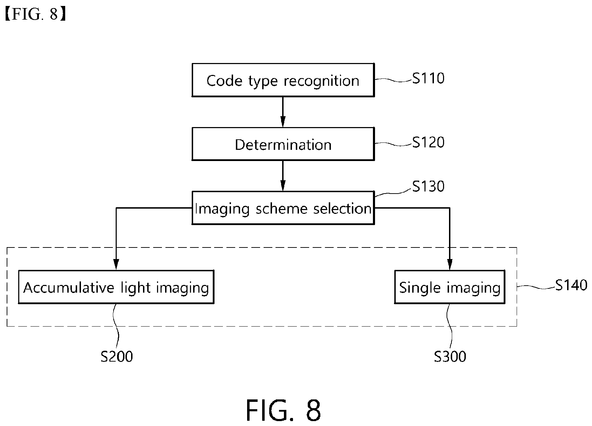

As illustrated in FIG. 8, the method for controlling a chromatographic testing device according to the present exemplary embodiment may include a code expression recognition step (S110), a determination step (S120), an imaging scheme selection step (S130) and an imaging step (S140).

As described above, the code expression recognition step (S110) is a step in which the code expression recognition unit 150 recognizes a code expression 230 of the immunochromatographic test cartridge 200 mounted on the cartridge tray 120 of the body 110.

The determination step (S120) is a step of determining information such as the type of fluorescent dye applied to the immunochromatographic test cartridge 200, as the information of the immunochromatographic test cartridge 200 recognized in the code expression recognition step (S1110).

For example, through the reading of the information described in the code expression 230 in the determination step (S120), it may be determined whether the fluorescent dye applied to the immunochromatographic test cartridge 200 is the time resolved fluorescence (TRF) method to which an organic phosphor is applied or the QD method to which quantum dots are applied.

Certainly, other types of fluorescent dyes may be applied in addition to the above.

The imaging scheme selection step (S130) is a step of selecting an excitation light imaging scheme according to the type of the fluorescent dye determined in the determination step (S120).

That is, if the fluorescent dye determined in the determining step (S120) is a time resolved fluorescence (TRF), the cumulative light imagine scheme (S200) may be selected in the imaging scheme selection step (S130), and if the fluorescent dye determined in the determination step is a quantum dot, the single imaging scheme (S300) may be selected in the imaging scheme selection step (S130).

The imaging step (S140) is a step of imaging the excitation light according to the imaging scheme selected in the imaging scheme selection step (S130).

That is, in the imaging scheme selection step (S130), when the cumulative light imaging scheme (S200) is selected, the excitation light is photographed by the corresponding scheme, and when the single imaging scheme (S300) is selected in the imaging scheme selection step (S130), the excitation light is photographed by the corresponding scheme.

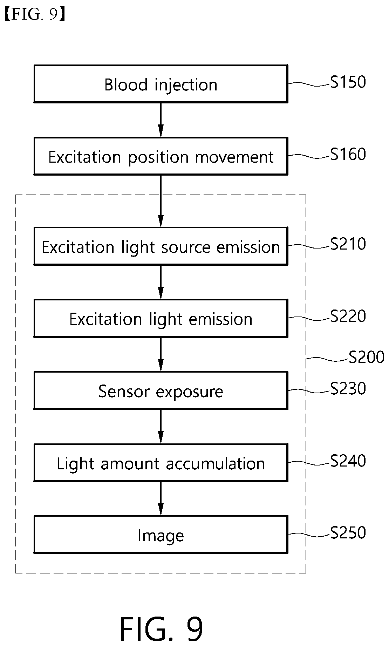

In this case, as illustrated in FIG. 9, the cumulative light imaging scheme (S200) may include an excitation light source emission step (S210), an excitation light emission step (S220), a light amount accumulation step (S240) and a cumulative light amount imaging step (S250).

The excitation light source emission step (S210) may be a step in which the excitation light source is emitted, and the excitation light emission step (S220) may be a step in which the excitation light is emitted from the immunochromatographic test cartridge by the emission of the excitation light source.

The light amount accumulation step (S240) is a step in which the image sensor unit 140 is exposed (S230) to receive the excitation light and the amount of light received is accumulated, after the background noise of the excitation light generated in the excitation light emission step (S220) is reduced.

In addition, the excitation light source emission step (S210), the excitation light emission step (S220) and the light amount accumulation step (S240) may be repeated for a set number of times, and the amount of light may be accumulated in the image sensor unit 140 during the repetition.

The cumulative light amount imaging step (S250) is a step of generating an image as the amount of light accumulated in the image sensor unit 140 while the excitation light source emission step (S210), the excitation light emission step (S220) and the light amount accumulation step (S240) are repeated.

As described above, as the emission of the excitation light source and the reception of the excitation light are repeated, electrons are accumulated in the image sensor unit 140, and a clearer image may be obtained in proportion to the repetition of the emission of the excitation light and the reception of the excitation light.

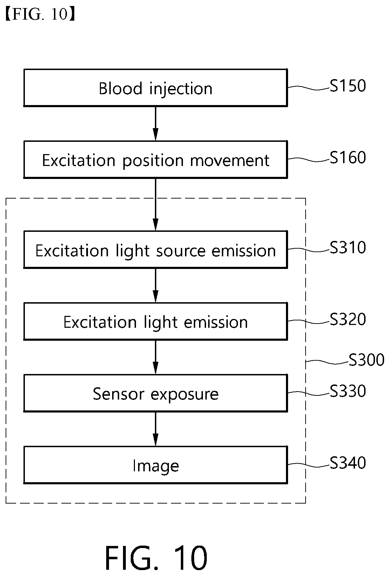

Meanwhile, as illustrated in FIG. 10, in the single imaging scheme (S300), an excitation light source emission step (S310), an excitation light step (S320), a sensor exposure step (S330) and a single light amount imaging step (S340) may be performed.

The excitation light source emission step (S310) and the excitation light emission step (S320) may be substantially identical to the excitation light source emission step (S210) and the excitation light emission step (S220) of the above-described cumulative light imaging scheme (S200). However, although the excitation light source emission step (S210), the excitation light emission step (S220), the sensor exposure (S230) and the light amount accumulation step (S240) are repeated for a set number of times in the above-described cumulative light imaging scheme (S200), the single imaging scheme (S300) differs in that the excitation light source emission step (S310) and the excitation light emission step (S320) are performed only once.

That is, the excitation light source emission step (S310) and the excitation light emission step (S320) are performed once, and accordingly, the image sensor unit 140 is exposed and the sensor exposure step (S330) for receiving the excitation light may also be performed only once.

Accordingly, the amount of light is not accumulated in the image sensor unit 140, and an image may be generated in a single light amount imaging step (S340) as the amount of light received once.

In this case, a blood injection step (S150) and an excitation position moving step (S160) may be performed.

The blood injection step (S150) is a step of dropping a sample into the immunochromatographic test cartridge 200. That is, the sample, such as blood, body fluid or the like, is dropped into the cartridge 200, and the sample may be dropped into the cartridge 200 through the injection hole 210 of the cartridge 200. In this case, the injection hole 210 of the cartridge 200 mounted on the cartridge tray 120 may be exposed at a place suitable for blood injection, such as the outside of the body 110.

The sample dropped into the cartridge 200 may move to the immune strip according to the internal structure of the cartridge 200 to cause an antibody-antigen reaction.

The excitation position moving step (S160) is a step of moving the immunochromatographic test cartridge 200 on which the blood injection has been completed to an excitation position for reading the excitation light. In this case, the excitation position may be a step in which the window 220 of the immunochromatographic test cartridge is located directly below the image sensor 142.

This blood injection step may be performed after or before the code expression recognition step (S1110).

In addition, the excitation position moving step (S160) may be performed after the blood injection step (S150) and the code expression recognition step (S110).

In the present exemplary embodiment, the blood injection step (S150) and the excitation position moving step (S160) have been described as being formed between the imaging scheme selection step (S130) and the imaging step (S140), but the present invention is not necessarily limited thereto, and these steps may be performed simultaneously with the determination step (S120) or the imaging scheme selection step (S130).

As described above, the preferred exemplary embodiments according to the present invention have been described, and the fact that the present invention can be embodied in other specific forms without departing from the spirit or scope of the present invention in addition to the above-described exemplary embodiments is apparent to those having ordinary skill in the art. Therefore, the above-described exemplary embodiments are to be regarded as illustrative rather than restrictive, and accordingly, the present invention is not limited to the above description, but may be modified within the scope of the appended claims and their equivalents.

## Claims

1. A chromatographic testing device, comprising:
a body;
a code expression recognition unit provided in the body and recognizing a code expression in which a type of fluorescent dye of an immune strip provided on an immunochromatographic test cartridge mounted on the body is recorded;
an excitation light source emission unit for emitting light from an excitation light source to the immunochromatographic test cartridge;
an image sensor unit for recognizing excitation light generated by the excitation light source;
and a control unit for controlling the image sensor unit in a single imaging scheme or a cumulative light imaging scheme according to the type of fluorescent dye of an immune strip, recognized by the code expression recognition unit.

2. The chromatographic testing device of claim 1, wherein the code expression recognition unit determines whether the fluorescent dye of the immunochromatographic test cartridge mounted on the body is a time resolved fluorescence (TRF) or a quantum dot through the code expression recognition.

3. The chromatographic testing device of claim 2, wherein the control unit controls the image sensor unit to photograph in a cumulative light imaging scheme, when the fluorescent dye of the immune strip recognized by the code expression recognition unit is a TRF, and controls the image sensor unit to photograph in a single imaging scheme, when the fluorescent dye of the immune strip recognized by the code expression recognition unit is a quantum dot.

4. The chromatographic testing device of claim 1, wherein in the cumulative light imaging scheme, the excitation light source emission unit emits light multiple times for a set number of times, and the image sensor unit is exposed to excitation light multiple times each time the excitation light source emission unit emits light, and the amount of light detected each time it is exposed to the excitation light is accumulated and imaged as an amount of light accumulated in the image sensor unit.

5. The chromatographic testing device of claim 1, wherein in the single imaging scheme, the excitation light source emission unit emits light a single time, and when the excitation light source emission unit emits light, the excitation light is received by the image sensor unit and imaged.

6. The chromatographic testing device of claim 1, wherein the code expression recognition unit comprises a code expression illumination light source for illuminating the code expression, and
wherein the image sensor unit receives light emitted from the code expression illumination light source and reflected in the code expression, and the control unit reads the code expression information.

7. A method for controlling a chromatographic testing device, comprising:
a code expression recognition step of recognizing a code expression of an immunochromatographic test cartridge mounted on a body;
a determination step of determining the type of fluorescent dye of the immunochromatographic test cartridge recognized in the code expression recognition step;
an imaging scheme selection step of selecting an excitation light imaging scheme according to the type of fluorescent dye determined in the determination step; and
an imaging step of photographing excitation light according to the imaging scheme selected in the imaging scheme selection step.

8. The method of claim 7, wherein the determination step determines whether the fluorescent dye of the immunochromatographic test cartridge is a time resolved fluorescence (TRF) or a quantum dot.

9. The method of claim 8, wherein when the fluorescent dye determined in the determination step is a time resolved fluorescence (TRF), a cumulative light imaging scheme is selected in the imaging scheme selection step.

10. The method of claim 8, wherein when the fluorescent dye determined in the determination step is a quantum dot, a single imaging scheme is selected in the imaging scheme selection step.

11. The method of claim 9, wherein the cumulative light imaging scheme comprises:
an excitation light source emission step in which an excitation light source emits light;
an excitation light emission step in which excitation light is emitted from the immunochromatographic test cartridge by an excitation light source;
a light amount accumulation step of accumulating an amount of light received while an image sensor unit is exposed to receive the excitation light, after a background noise of the excitation light generated in the excitation light emission step is reduced; and
a cumulative light amount imaging step of generating an image as an amount of light accumulated in the image sensor unit during which the excitation light source emission step, the excitation light emission step and the light amount accumulation step are repeated for a set number of times.

12. The method of claim 10, wherein the single imaging scheme comprises:
an excitation light source emission step in which an excitation light source emits light;
an excitation light emission step in which excitation light is emitted from the immunochromatographic test cartridge by an excitation light source;
a sensor exposure step in which the image sensor unit is exposed to receive the excitation light; and
a single light amount imaging step of generating an image with the amount of light received in the sensor exposure step.

13. The method of claim 7, further comprising:
a blood injection step for dropping a sample into the immunochromatographic test cartridge; and
an excitation position moving step for moving the immunochromatographic test cartridge on which the blood injection has been completed to an excitation position for reading excitation light.
